(19) **Europäisches Patentamt / European Patent Office / Office européen des brevets**

(11) **EP 4 233 863 A1**

(12) **EUROPEAN PATENT APPLICATION**
published in accordance with Art. 153(4) EPC

(43) Date of publication:
**30.08.2023 Bulletin 2023/35**

(21) Application number: **21886643.2**

(22) Date of filing: **15.10.2021**

(51) International Patent Classification (IPC):
**A61K 31/4178** (2006.01)   **A61K 39/395** (2006.01)
**A61K 51/10** (2006.01)   **A61P 35/00** (2006.01)
**C07K 16/32** (2006.01)   **A61K 39/00** (2006.01)

(52) Cooperative Patent Classification (CPC):
**A61K 31/4178; A61K 39/00; A61K 39/395;
A61K 51/10; A61P 35/00; C07K 16/32**

(86) International application number:
**PCT/KR2021/014360**

(87) International publication number:
**WO 2022/092649 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 26.10.2020   KR 20200139432
13.10.2021   KR 20210135651

(71) Applicant: **Korea Institute of Radiological &
Medical Sciences**
**Seoul 01812 (KR)**

(72) Inventors:
• **KIM, Jin Su**
**Seoul 01816 (KR)**
• **KIM, Hyeongi**
**Seoul 02007 (KR)**
• **ZAHEER, Javeria**
**Seoul 01836 (KR)**

(74) Representative: **Roos, Peter**
**c/o Roospatent**
**Noldinstrasse 7**
**81545 München (DE)**

(54) **COMPOSITION FOR ENHANCING TUMOR PENETRATION OF ANTICANCER DRUG,
COMPRISING LOSARTAN AS ACTIVE INGREDIENT, AND USE THEREOF**

(57)     The present invention relates to a composition for enhancing tumor penetration of an anticancer drug, comprising losartan as an active ingredient, and a use thereof. In the composition, losartan decomposes collagen and hyaluronic acid, which are main components of the extracellular matrix, to enhance the penetration of various anticancer drugs, including an antibody for cancer treatment, into the center of a tumor and uniformly distribute the various anticancer drugs, thereby improving a cancer treatment effect of an anticancer drug. Therefore, according to the present invention, by using combination therapy of losartan and an anticancer drug including an antibody for cancer treatment, penetration of the anticancer drug into the center of tumor can be enhanced, thereby making it possible to treat cancer more effectively.

[FIG. 1]

EP 4 233 863 A1

**Description**

Technical Field

The present disclosure relates to a composition for enhancing tumor penetration of an anticancer drug, including losartan as an active ingredient, and a use thereof.

Background Art

Gastric cancer is one of main types of cancers worldwide, and its risk varies depending on heterogeneity, smoking, and environmental factors. According to data from the Global Burden of Disease Study in 2017, there were 1.22 million cases of gastric cancer worldwide, resulting in an estimated 848,000 to 885,000 deaths.

Radioimmunotherapy (RIT) is a therapy that maximizes treatment effects by labeling therapeutic radioactive isotopes that emit beta rays (I-131, Y-90, Lu-177, etc.) or alpha particles (At-211, Ac-225, Ra-223, Bi-213, etc.) with monoclonal antibody (mAb) specific for tumor epitopes for combined use of anticancer effects of antibodies and radiation that exhibits cytotoxicity.

Drugs for radioimmunotherapy that are currently in use in clinical practice include tositumomab (Bexxar), ibritumomab tiuxetan (Zevalin), and rituximab (Rituxan) for treatment of hematologic tumor as well as trastuzumab (Herceptin), cetuximab (Erbitux), and panitumumab (Vectibix) for treatment of solid tumors.

However, RIT shows insufficient dose delivery in solid tumors with low reactivity due to radiation resistance of solid tumors, despite being effective in hematologic tumors. Limited targeting and insufficient dose delivery of mAbs for solid tumors are due to factors such as high extracellular matrix (ECM), abnormal structures of tumor blood vessels, tumors forming highly fibrous or connective tissues, absence of functional lymph nodes, and high pressure inside tumors. For this reason, due to limited penetration of antibodies into tumors, the clinical application of RIT for solid tumors is cost-ineffective and does not show satisfactory treatment outcome.

Furthermore, in the case of Y-90 and Lu-177, which are used as therapeutic radiation isotopes, the maximum radiation range is 12.9 mm and 2.1 mm, respectively, which may cause radiation exposure of surrounding normal tissues if the antibody fails to penetrate the center of the tumor.

Therefore, there is a need for new technologies that may improve tumor penetration of anticancer drugs such as antibodies to treat cancer more effectively.

Disclosure of the Invention

Technical Goals

An object of the present disclosure is to provide a composition for enhancing tumor penetration of an anticancer drug.

Another object of the present disclosure is to provide a combined therapy for treating cancer diseases to enhance tumor penetration of an anticancer drug.

Technical Solutions

In order to achieve the above object, the present disclosure provides a composition for enhancing tumor penetration of an anticancer drug, including losartan as an active ingredient.

The present disclosure provides a pharmaceutical composition for treating a cancer disease, including losartan and any one anticancer drug of a chemo-anticancer drug, a targeted anticancer drug, or an immuno-anticancer drug labeled or unlabeled with radioisotopes. Advantageous Effects

A composition according to the present disclosure may promote penetration of various anticancer drugs including antibodies for cancer treatment into the center of tumor as losartan degrades collagen and hyaluronic acid, which are main components of the extracellular matrix, and uniformly distributes the anticancer drugs to enhance a cancer treatment effect of the anticancer drug.

Therefore, according to the present disclosure, it is possible to derive an outstanding anticancer effect than the sole treatment of anticancer drugs through a combined therapy of losartan and the anticancer drug including antibodies for cancer treatment.

Brief Description of Drawings

FIG. 1 schematically illustrates a tumor penetration enhancement effect of antibodies using trastuzumab (Cu-64-TRZ) labeled with radioactive isotopes (Cu-64) and losartan.

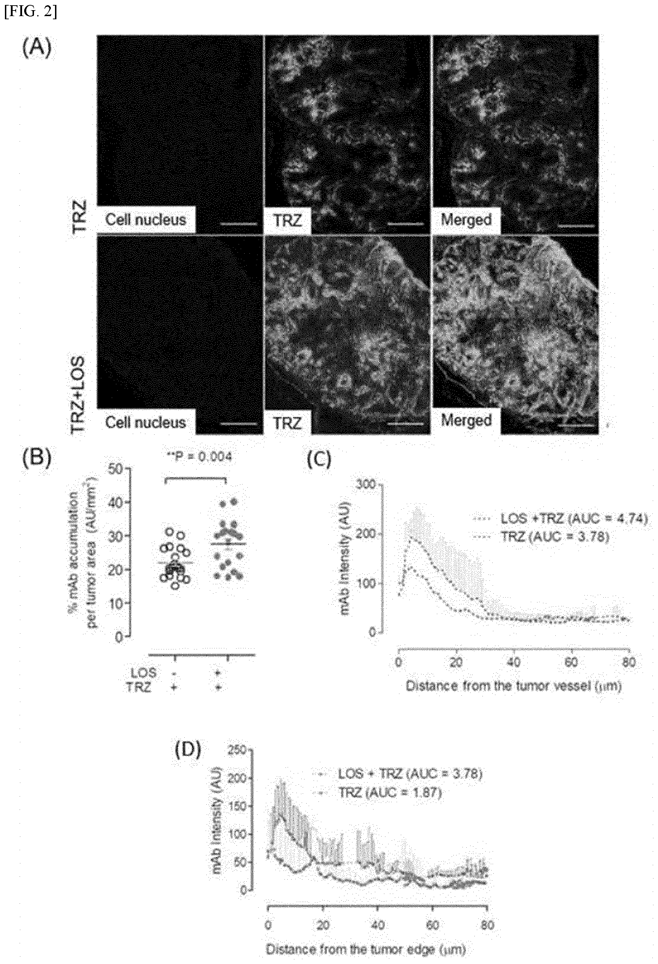

FIG. 2 shows comparison of distribution in gastric tumor tissues upon sole treatment of trastuzumab and co-treatment of losartan and trastuzumab, wherein (A) is representative fluorescence images obtained 5 days after injection of Alexa-647-TRZ in an NCI-N87 tumor xenograft model, where DAPI is in blue, TRZ (Alexa-647-TRZ) in green, and blood vessels of TRZ alone (vessel and edge image at the top) and TRZ + losartan (TRZ + LOS) group (vessel and edge image at the bottom) in red (lectin), (B) is a result of quantitatively analyzing TRZ (green signal) across tumor tissue sections in each group via MIPAV software that plots a region of interest over the entire tumor tissue section, represented by a scatter bar graph with mean lines having error bars in standard deviations (SD) (** $P < 0.005$), and (C) and (D) are results of quantitative analysis on accumulation of TRZ and TRZ + losartan from tumor blood vessels and tumor edges, represented by drawing volume of interest (VOI) lines from the vicinity of tumor blood vessels and tumor edges using the MIPAV program. The generated histogram is saved as a pit file which is read using an in-house program written in MATLAB. Distances are measured up to a maximum length of 80 $\mu$m. The data are statistically significant, and error bars are displayed above each data point as standard deviations.

FIG. 3 shows a degrading effect of the extracellular matrix (collagen, hyaluronic acid) upon treatment of losartan, wherein (A) shows collagen stained in blue in a control and losartan treated samples using a masson trichrome staining kit, and (B) shows hyaluronan stained in blue in the control and losartan treated samples using an alcian blue staining kit (magnification: x12.5, x100, and x400).

FIG. 4 shows PET/CT images of trastuzumab labeled with a radioactive isotope (Cu-64) in gastric cancer, wherein (A) shows a result of observing changes in intervals of 4h, 12h, 48h, 72h, 96h, and 120h after injection of $^{64}$Cu-TRZ (top, Cu-64 TRZ) and $^{64}$Cu-TRZ in combination with losartan (bottom, Cu-64 TRZ + LOS) after injection at 0 hour, and (B) shows a result of calculating a standard uptake value (SUV) in the PET image while the maximum SUV is evaluated at each time point (n = 6) for up to 5 days (* $P < 0.05$).

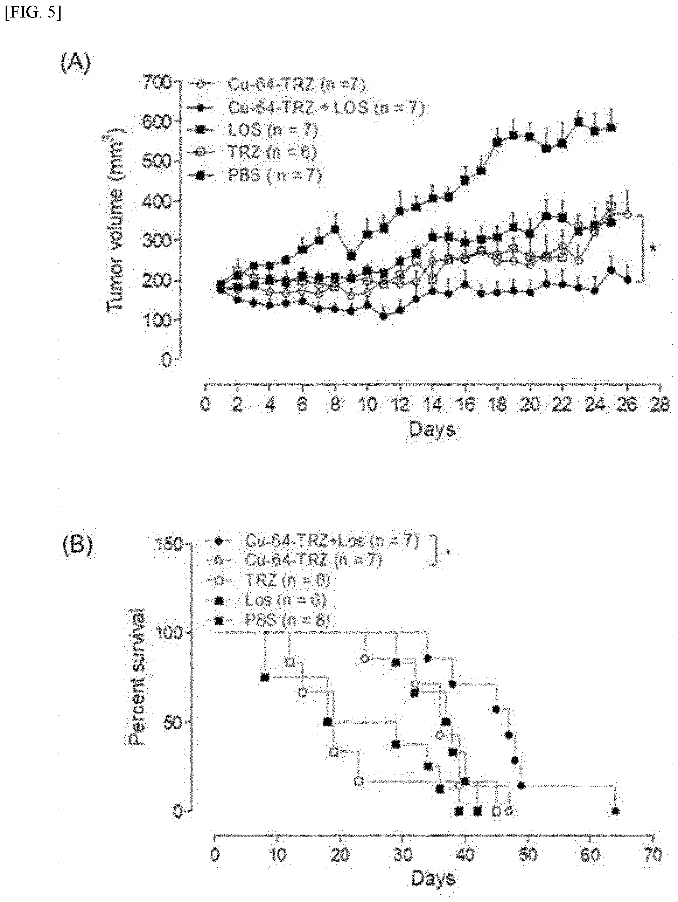

FIG. 5 shows results of evaluating a treatment effect of combined RIT of losartan and $^{64}$Cu-TRZ, wherein (A) shows a daily monitoring result of tumor growth for 25 days, drawn on a graph with error bars and standard deviations on each data (* $P < 0.05$), and (B) shows a result of plotting a survival rate with a Kaplan-Meier graph using graph pad version 5.02 after recording the survival of all groups of PBS, LOS, TRZ, $^{64}$Cu-TRZ (Cu-64-TRZ), and $^{64}$Cu-TRZ in combination with losartan (Cu-64-TRZ + LOS). The data are statistically significant ($^{64}$Cu-TRZ versus $^{64}$Cu-TRZ + LOS: * $P < 0.05$), (PBS versus $^{64}$Cu-TRZ: * $P < 0.05$), (PBS versus LOS: * $P < 0.05$).

FIGS. 6 to 8 show images identifying a tumor penetration improvement effect of cetuximab by losartan in tissues of brain tumor, lung cancer, and colorectal cancer, respectively.

FIGS. 9 to 12 show images identifying a tumor penetration improvement effect of atezolizumab by losartan in tissues of pancreatic cancer, brain tumor, lung cancer, and colorectal cancer, respectively.

FIG. 13 shows an image identifying a tumor penetration improvement effect of rituximab by losartan in B-cell lymphoma tissues.

FIG. 14 shows a tumor growth inhibitory effect by co-treatment of irinotecan and losartan in tissues of colorectal cancer.

Best Mode for Carrying Out the Invention

Hereinafter, the present disclosure will be described in detail.

As an example embodiment, the present inventor completed the present disclosure by determining that losartan degrades hyaluronic acid and collagen in the extracellular matrix (ECM) of gastric cancer tissues in NCI-N87 tumor xenograft mouse models to promote intratumoral penetration of $^{64}$Cu-trastuzumab (TRZ) so thus efficacy of radioimmunotherapy (RIT) was enhanced, identifying that losartan promotes intratumoral penetration of cetuximab, atezolizumab, and rituximab in A549 (lung cancer), U87MG (brain tumor), and Raji (B-cell lymphoma) xenograft mouse models, and first revealing that losartan promotes intratumoral penetration of cetuximab and atezolizumab in Hap-T 1 (pancreatic cancer) and CT26 (colorectal cancer) allograft mouse models and also promotes intratumoral penetration of irinotecan in CT26 (colorectal cancer) allograft mouse models.

Accordingly, the present disclosure provides a composition for enhancing tumor penetration of an anticancer drug, including losartan as an active ingredient.

The term "tumor penetration enhancement of an anticancer drug" as used herein refers to an ability of an anticancer drug that may break through physical and/or physiological barriers present around the tumor and travel closer to the center of tumor tissues. In the case of tumor tissues, it is difficult for the anticancer drug to penetrate into the tumor due to collagen formed around the tumor, abnormal blood vessel formation, and increased pressure between cells and tissues inside the tumor, but the composition of the present disclosure resolves the problem to allow the anticancer drug to better penetrate into the tumor.

According to an example embodiment of the present disclosure, when losartan is used in combination with an anticancer drug selected from antibodies or compounds, losartan degrades the extracellular matrix composed of collagen or hyaluronic acid to promote penetration of the anticancer drug into the tumor tissue and uniformly distributes the anticancer drug to enhance a treatment effect on the tumor.

Preferably, the composition may be a pharmaceutical composition for assisting anticancer treatment to enhance tumor penetration of the anticancer drug.

Preferably, the composition may be a pharmaceutical composition for assisting radioimmunotherapy that is used in combination with the anticancer drug in the radioimmunotherapy to enhance tumor penetration of the anticancer drug.

The anticancer drug may be any one of a chemo-anticancer drug, a targeted anticancer drug, or an immuno-anticancer drug labeled or unlabeled with radioisotopes.

The radioisotopes may be selected from the group consisting of $^{64}$Cu, $^{67}$Cu, $^{47}$Sc, $^{77}$As, $^{90}$Y, $^{109}$Pd, $^{111}$Ag, $^{131}$I, $^{142}$Pr, $^{149}$Pm, $^{153}$Sm, $^{159}$Gd, $^{166}$Ho, $^{177}$Lu, $^{186}$Re, $^{188}$Re, $^{194}$Ir, $^{199}$Au, $^{211}$At, $^{212}$Bi, $^{213}$Bi, $^{212}$Pb, $^{225}$Ac, and $^{227}$Th, but are not limited thereto.

The chemo-anticancer drug may be selected from the group consisting of abemaciclib, everolimus, alpelisib, anastrozole, pamidronic acid, exemestane, capecitabine, eribulin, everolimus, exemestane, toremifene, olaparib, palbociclib, thiotepa, irinotecan, gemcitabine, oxaliplatin, bortezomib, cisplatin, paclitaxel, gefitinib, lapatinib, sorafenib, 5-FU (5-fluorouracil), and doxorubicin, but is not limited thereto.

The targeted anticancer drug may be selected from the group consisting of abciximab, adalimumab, basiliximab, bezlotoxumab, canakinumab, daclizumab, denosumab, efalizumab, golimumab, inflectra, natalizumab, olaratumab, omalizumab, palivizumab, panitumumab, trastuzumab, pertuzumab, cetuximab, rituximab, tocilizumab, secukinumab, ustekinumab, bevacizumab, and avelumab, but is not limited thereto.

The immuno-anticancer drug may be selected from the group consisting of an anti-PD-1 antibody, an anti-PD-L1 antibody, and an anti-CTLA-4 antibody, but is not limited thereto.

The immuno-anticancer drug may be selected from the group consisting of pembrolimab, nivolumab, atezolizumab, durvalumab, and ipilimumab, but is not limited thereto.

The antibody for the anticancer drug is an antibody that exhibits anticancer activity by binding to a cancer-related antigen that is associated with cancer formation, cancer progression, development or death, and may include not only a complete antibody form, but also an antigen binding fragment (antibody fragment) of an antibody molecule.

The antibody for the anticancer drug may be a monoclonal antibody, but is not limited thereto.

As an example embodiment, the antibody for the anticancer drug may be an antihuman epidermal growth factor receptor 2 (HER2) protein, and may include a combination (ex. Phesgo) of trastuzumab (Herceptin), pertuzumab (Perjeta), or one or more thereof and hyaluronidase, but is not limited thereto.

In addition, as another example embodiment, it may include, ado-trastuzumab emtansine (Kadcyla) and fam-trastuzumab deruxtecan (Enhertu) which are combination of anti-HER2 antibodies and drugs, but is not limited thereto.

In addition, the present disclosure provides a pharmaceutical composition for treating a cancer disease, including losartan and any one anticancer drug of a chemo-anticancer drug, a targeted anticancer drug, or an immuno-anticancer drug labeled or unlabeled with radioisotopes.

Herein, the cancer disease may be solid cancer or hematologic tumor, preferably gastric cancer, lung cancer, brain tumor, lymphoma, pancreatic cancer, colon cancer, or breast cancer, but is not limited thereto.

The corresponding features are the same as described above, and the description is omitted.

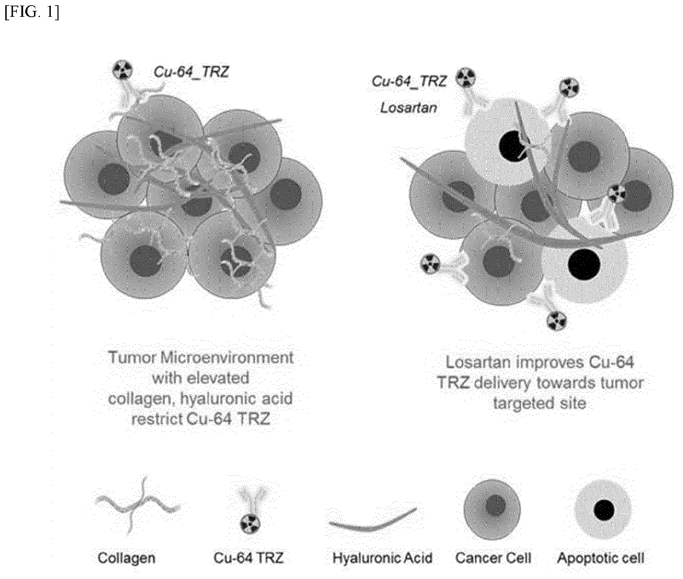

Referring to the drawings, when losartan is added along with trastuzumab (Cu-64-TRZ) labeled with a radioactive isotope (Cu-64), it was expected that radioimmunotherapy (RIT) efficacy in gastric cancer may be enhanced as shown in FIG. 1, by degrading and regulating the extracellular matrix (ECM) such as collagen and hyaluronic acid, as shown in FIG. 2, losartan improved tumor penetration of trastuzumab throughout tumor tissues, and the tumor growth of $^{64}$Cu-TRZ was inhibited by the combined use of losartan as shown in FIG. 5.

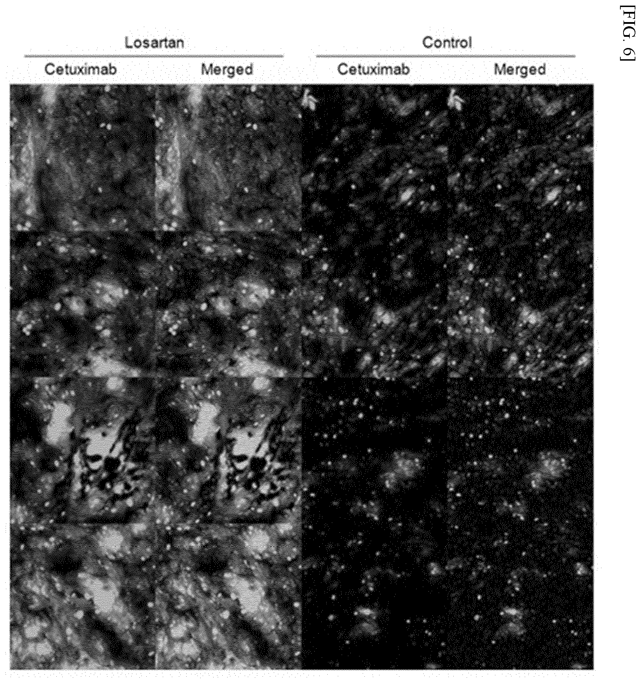

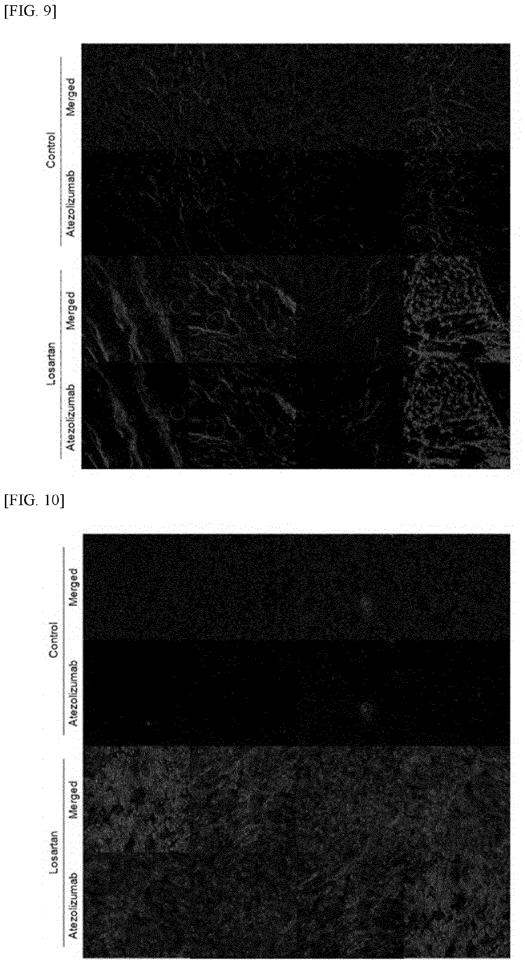

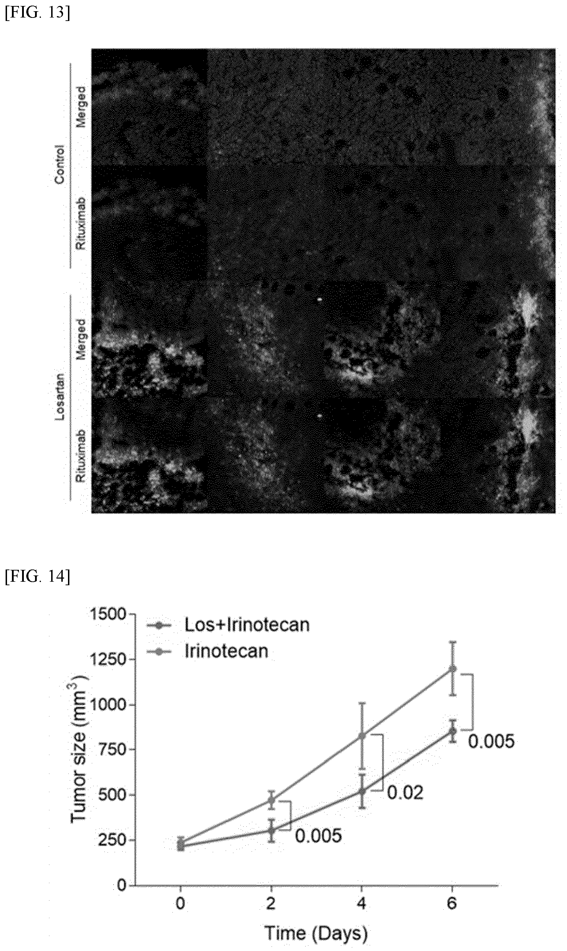

In addition, according to FIGS. 6 to 8, losartan improved tumor penetration of cetuximab throughout tissues of brain tumor, lung cancer, and colorectal cancer, according to FIGS. 9 to 12, losartan improved tumor penetration of atezolizumab throughout tissues of lung cancer, brain tumor, pancreatic cancer, and colorectal cancer, and according to FIG. 13, losartan improved tumor penetration of rituximab in tissues of B cell lymphoma. Moreover, according to FIG. 14, losartan improved tumor penetration of irinotecan in tissues of colorectal cancer, and tumor growth was inhibited by combined therapy.

The pharmaceutical composition according to the present disclosure may be prepared according to conventional methods in the pharmaceutical field. The pharmaceutical composition may be blended with an appropriate pharmaceutically acceptable carrier according to the formulation, and may be prepared, as needed, by further including excipients, diluents, dispersants, emulsifiers, buffers, stabilizers, binders, disintegrants, and solvents. The appropriate carrier does not interfere with the activity and properties of the composition according to the present disclosure and may be selected differently according to the dosage form and formulation.

The carrier, excipient, and diluent that may be included in the pharmaceutical composition may include lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil. When the composition is formulated, it may be prepared using diluents or excipients such as fillers, extenders, binders, wetting agents, disintegrants, and surfactants that are commonly used.

The pharmaceutical composition according to the present disclosure may be applied in any formulation, and more specifically, it may be used by formulating in a form of oral formulations such as acids, granules, tablets, capsules, suspensions, emulsions, syrups, and aerosols, external agents, suppositories, and sterile injection solutions according to conventional methods.

More specifically, the solid formulation of the oral formulation is in the form of tablets, pills, acids, granules, and capsules, preparation may be performed by mixing at least one or more excipients, such as starch, calcium carbonate, sucrose, lactose, sorbitol, mannitol, cellulose, and gelatin, and lubricants such as magnesium stearate and talc may also be included in addition to simple excipients. In addition, in the case of the capsule formulation, a liquid carrier such as fatty oil may be further included in addition to the above-mentioned substances.

The liquid formulation of the oral formulation includes suspensions, solutions, emulsions, and syrups, and various excipients such as wetting agents, sweeteners, fragrances, and preservatives may be included in addition to commonly used simple diluents such as water and liquid paraffin.

The parenteral formulation may include a sterilized aqueous solution, a non-aqueous solvent, a suspending agent, an emulsion, an injection, a lyophilized preparation, and a suppository. Propylene glycol, polyethylene glycol, vegetable oils such as olive oil, and injectable esters such as ethyl oleate may be used as non-aqueous solvents and suspending agents. Witepsol, macrogol, Tween 61, cacao butter, laurin fat, and glycerogelatin may be used as a base of the suppositories. Without limitation, all suitable preparations well known in the art may be used.

The pharmaceutical composition according to the present disclosure may be administered in a pharmaceutically effective amount.

The term "pharmaceutically effective amount" as used herein refers to an amount sufficient to treat a disease at a reasonable benefit/risk ratio applicable to medical treatment without causing adverse effects.

The effective dose level of the pharmaceutical composition may be variably determined by the purpose of use, the age, sex, body weight, and health condition of a patient, type of disease, severity, activity of a drug, sensitivity to a drug, administration methods, administration duration, administration route and discharge rate, treatment periods, factors including drugs used in combination or simultaneously, and other factors well known in the medical field. For example, although not constant, the administration may be performed generally at 0.001 to 100 mg/kg, preferably 0.01 to 10 mg/kg once to several times daily. The dosage does not limit the scope of the disclosure in any way.

The pharmaceutical composition according to the present disclosure may be administered to any animal in which cancer may occur, and the animal may include, for example, not only humans and primates, but also livestock such as cattle, pigs, horses, and dogs.

The pharmaceutical composition may be administered by a suitable administration route according to the formulation form and administered through various oral or parenteral routes as long as it may reach a target tissue. Administration methods need not be particularly limited, for example, administration may be performed in conventional methods such as oral, rectal or intravenous, intramuscular, skin application, respiratory inhalation, intrauterine dural or intracere-broventricular injection.

The pharmaceutical composition according to the present disclosure may be solely used for cancer treatment, used in combination with surgery or other drug treatment, and administered sequentially or simultaneously with conventional treatment agents.

Modes for Carrying Out the Invention

Hereinafter, example embodiments will be described in detail to help the understanding of the present disclosure. However, the following example embodiments are merely illustrative of the content of the present disclosure, and the scope of the present disclosure is not limited to the following examples. The example embodiments of the present disclosure are provided to more completely explain the present disclosure to those skilled in the art.

<Examples>

1. Experimental Method

1-1. Animal care

All animal tests were conducted in accordance with the guidelines of Korea Institute of Radiological & Medical Sciences (KIRAMS) and approved by Institutional Animal Care and Use Committee (IACUC number: KIRAMS 2020-0027). BALB/c nude mice (Shizuoka laboratory center, Japan) were 5 to 6 weeks old at the start of the experiment. The animals were weighed and kept in cages for 24 hours during the day and night cycle.

1-2. Cell culture

NCI-N87 (HER2-positive) gastric cancer cell lines, A549 (EGFR-positive and PD-L1 positive) lung cancer cell lines, U87MG (EGFR-positive and PD-L1 positive) brain tumor cell lines, and Raji (CD20-positive) B cell lymphoma cell lines were obtained from the American Type Culture Collection (ATCC). CT26 (EGFR-positive and PD-L1-positive) colorectal cancer cell lines were obtained from Korea Cell Line Bank. The HaP-T1 pancreatic cancer cell lines were obtained from Dr. Masato Abei (University of Tsukuba, Ibaraki, Japan).

Culture media were formed of 5% antibiotics (Sigma, St. Louis, MO, USA) and 10% fetal bovine serum. Cells in the prepared media including Roswell Park Memorial Institute (RPMI) or Dulbecco's modified Eagle's medium (DMEM) were maintained at 37°C in a humid 5% $CO_2$ incubator.

1-3. Xenograft models and allograft (syngeneic) models

For tumor xenograft, preparation was performed by subcutaneous injection of NCI-N87, A549, or U87MG cells ($5 \times 10^6$) according to 1-2 into BALB/c nude mice, and subcutaneous injection of Raji cells ($1 \times 10^7$) into SCID mice.

The allograft model was prepared by subcutaneous injection of CT26 cells ($5 \times 10^6$) according to 1-2 into BALB/c mice. The allograft model was prepared by subcutaneous injection of Hap-T1 cells ($3 \times 10^6$) according to 1-2 above into golden hamster. Tumor growth was monitored, and tumor volume was calculated using the following equation: $Width^2$ x Length x 0.5

1-4. Preparation of Alexa 647 (or 488)-antibodies

Alexa Fluor 647 (or 488) NHS Ester (1 mg, Invitrogen, USA) solution dissolved in 155 $\mu$l of dimethyl sulfoxide (DMSO) containing 1% acetic acid was mixed immediately with 500 $\mu$l of trastuzumab (Roche, USA, 10 mg/ml in PBS), rituximab (Roche, USA, 10 mg/ml in PBS), cetuximab (Merck, USA, 10 mg/ml in PBS), or atezolizumab (Roche, USA, 10 mg/ml in PBS), and 250 $\mu$l of 1M pH 8.5 sodium bicarbonate solution. The solution was cultured at room temperature for 1 hour. Alexa 647 (or 488)-antibodies were purified by a size-exclusion PD-10 column (GE Healthcare Bio-Sciences AB, Sweden) and eluted with phosphate-buffered saline (PBS). The molecular level of Alexa 647 (or 488) conjugated per antibody was evaluated using the Alexa 647 peak intensity distributed between mAb-Alexa 647 (or 488) conjugate and free Alexa-488 eluted from the size-exclusion HPLC.

1-5. Analysis on antibody penetration

Grouping for infiltration and distribution studies consisted of two groups of five mice each. Groups 1 and 2 were administered with 150 $\mu$g of fluorescently labeled antibodies (trastuzumab, rituximab, cetuximab, atezolizumab) conjugated to Alexa 647 (or 488) including or not including losartan, with subcutaneous injection involved by 60 mg/kg for 5 days.

When the tumor size reached 200 $\pm$ 20 $mm^3$, fluorescently labeled antibodies (150 $\mu$g in 100 $\mu$l of PBS) were injected intravenously into tumor-bearing mice (or hamsters), and losartan (40 mg/kg) was injected for 5 days (subcutaneous injection for hamsters). Five days after injection of the fluorescently labeled antibody, mice (or hamsters) were euthanized by injecting $CO_2$, and blood was collected through cardiac puncture before dissection.

1-6. Preparation of tissue slides for analysis of antibody distribution

The tumor was isolated and fixed overnight at 4°C with 4% paraformaldehyde. The tumor was dehydrated with 30% sucrose that was dissolved in PBS solution until the tissue sank to the bottom of a tube at 4°C and froze in OCT compounds (Sakura Finetec, USA). Frozen tumors were subjected to sectioning in a thickness of 8 $\mu$m using cryostat microtome (CM1800; LEICA Instruments) at different cross-sectional areas across the tumor (25%, 50%, and 75% at the tumor apex).

Then, seven sections were randomly selected from each cross-sectional area of three different tumors (n = 3). The tumor sections were fixed with 4% paraformaldehyde for 10 minutes, washed three times with PBS for 5 minutes, and then mounted with Prolong Gold antifade reagent (Invitrogen, USA) containing 4, 6-diamidino-2-phenyindole (DAPI).

1-7. Collection and analysis of fluorescence images

Fluorescence images were acquired with a 10x objective lens using an IN cell analyzer (IN cell analyzer 2200, GE Healthcare, Uppsala, Sweden) equipped with mosaic stitching software (in cell developer toolbox, GE Healthcare). Three independent channels were acquired as follows:

For NIC-N87, DAPI for nuclei (shown in blue, Ex/Em=358/461 nm), rhodamine for blood vessels (shown in red, Ex/Em = 558/581 nm), and Cy5 for Alexa 647-TRZ (shown in green, Ex/Em = 594/633 nm).

For U87MG, Raji, A549, CT26, and HapT1, DAPI for nuclei (shown in blue, Ex/Em=358/461 nm), Alexa 488-cetuximab or Alexa 488-rituximab (shown in green, Ex/Em = 495/519 nm), and Cy5 for Alexa 647-atezolizumab (shown in red, Ex/Em = 594/633 nm).

Image analysis was performed using in-house programs written in MATLAB (Math Works, Natick, MA, USA), ZEN (blue) and MIPAV (National Institutes of Health, Bethesda, MD, USA). Briefly, a TIFF file was exported and read as a line graph plotting using MIPAV software. TRZ intensity was calculated by reading images from the marginal blood vessels with volume of interest (VOI) drawn. Total accumulation was calculated by plotting the region of interest (ROI) over the entire tumor. The area under curve (AUC) was calculated and shown on the graph, and the total Alexa 647-TRZ accumulation was calculated by the following:

$$\text{Total tumor intensity of mAb} = \text{intensity of mAb in tumor/total tumor area}$$

1-8. Masson trichrome staining

Masson trichrome staining was performed for histological visualization of collagen, an extracellular component, which is considered as a disorder when TRZ travels. Tumor tissues were obtained by sacrificing animals. The obtained tissues were fixed overnight in 4% paraformaldehyde and transferred to OCT compounds (Sakura Finetec, USA). The tissues were sectioned to a thickness of 40 $\mu$m. Staining was performed according to the kit protocol (Abcam (www.abcam.com) ab150686).

Bouin's Fluid was applied to each section and cultured overnight at room temperature. The slides were washed with water, cultured in Weigert's iron hematoxylin for 5 minutes, then washed with tap water, and cultured in Biebrich scarlet/acid fuchsin solution for 15 minutes. Without washing, the slides were re-cultured with aniline blue for 30 minutes. The slides were then washed with water and cultured in acetic acid for 3-5 minutes.

It was also dehydrated in graded alcohol, washed in xylene, and then mounted to a class slide using a mounting medium. The slides were visualized, and images were filmed with the Olympus BX53 system microscope (Olympus, life science solution, Waltham, Massachusetts (02453)).

1-9. Hyaluronan-staining

Hyaluronan staining was performed using alcian blue stain kit (Vector laboratories, Burlingame, CA 94010, Alcian Blue (pH 2.5), Stain kit, H-3501). The tissues were sectioned to a thickness of 20 $\mu$m. The sections were washed with water to remove excess OCT. Acetic acid was treated to tissue sections for 3 minutes, and then Alcian blue solution was treated at 37°C for 30 minutes. Excess staining was removed by washing with acetic acid. It was then washed with tap water for 2 minutes and then with distilled water for 1-2 minutes.

In addition, the nuclear fast red solution was treated for 5 minutes, and washing was followed with tap water and then with distilled water. The slides were dehydrated in graded alcohol and removed from xylene, and then a cover slip was mounted. The slides were visualized, and images were filmed with Olympus BX53 system microscope (Olympus, life science solution, Waltham, Massachusetts (02453)).

1-10. Radiolabeling of $^{64}$Cu-DOTA-trastuzumab

For PET scanner, a positron emitter was used to produce images of radionuclides produced using cyclotron irradiation with a medium half-life ($t_{1/2}$, 12.7 h) that $^{64}$Cu attenuates both $\beta$+ (655 keV, 17.4%) and $\beta$- (573 keV, 39.0%) emissions, which are suitable for mAbs labeling for RIT of cancer and PET images.

In this experimental example, $^{64}$Cu-DOTA-TRZ was used for both immuno-PET and -RIT. TRZ (20 mg) was mixed with 1 mg of DOTA-NHS-Ester (Macrocyclics, USA) in pH 8.5, 0.1 M sodium bicarbonate buffer solution. Conjugates were lightly shaken overnight at 4°C and separated from the un-conjugated chelate using a PD-10 column eluted in pH 6.5, 1 mM sodium acetate buffer solution. $^{64}$CuCl$_2$ (370 MBq) was added to 1 mg of DOTA-TRZ dissolved in 1 mM sodium acetate (pH 5.5) and cultured at 37°C for 1 hour. $^{64}$Cu-DOTA-TRZ was purified using a PD-10 column eluted with 1 mM sodium acetate buffer solution. According to ITLC analysis, the radiochemical purity was $\geq$ 98%.

1-11. PET/CT images of $^{64}$Cu-DOTA-trastuzumab

PET/CT images were obtained by Siemens Inveon, PET scanner (Siemens Medical Solutions, USA). When the tumor size reached a group average of 150-200 mm$^3$, $^{64}$Cu-DOTA-TRZ (150 $\mu$g, 3.7-7.4 MBq/ 200 $\mu$l) was injected intravenously in the presence or absence of losartan (60 mg/kg/day for 25 days). PET/CT scanning was performed at 4 hours and 12 hours on day 2, 3, 4 and 5. The mice were anesthetized using 2.5% isoflurane, and PET data was collected at a 15-minute scan time by an energy window of 350-650 keV. The OSEM2D reconstruction algorithm was applied to reconstruct the PET data in a matrix size of 128x128x159. The voxel size was 0.776 mm x 0.776 mm x 0.796 mm. CT images were obtained 15 minutes after PET with full rotation and 180° projections using the Inveon system. Inveon Research Workflow software was used for image visualization and quantification.

1-12. Analysis on treatment efficacy

1) Combined therapy using losartan and trastuzumab

To identify gastric cancer (NCI-N87) treatment efficacy of the combined therapy using losartan and trastuzumab, the groups were classified into $^{64}$Cu-TRZ treated group (n = 7), losartan-included $^{64}$Cu-TRZ treated group (n = 7), losartan solely treated group (n = 6), TRZ solely treated group (n = 6), and PBS treated group (n = 8). The treatment plan included 3.7~7.4 MBq/150 $\mu$g of $^{64}$Cu-TRZ in the presence or absence of losartan (60 mg/kg). Losartan was delivered subcutaneously (60 mg/kg for 25 days), and TRZ (150 $\mu$g) was injected intravenously once at the start of treatment.

2) Combined therapy using losartan and irinotecan

To identify colorectal cancer (CT26) treatment efficacy of the combined therapy using losartan and irinotecan, groups were classified into irinotecan treated group (n = 4) and losartan-included irinotecan treated group (n = 4). The treatment plan included 20 mg/kg of irinotecan in the presence or absence of losartan (60 mg/kg). Losartan was delivered subcutaneously (60 mg/kg for 5 days), and irinotecan (20 mg/kg) was injected intravenously once at the start of treatment.

2. Experimental Results

2-1. Identification of tumor penetration improvement of trastuzumab by losartan

First, evaluation was performed on microdistribution of TRZ over gastric cancer tissues with losartan. Due to high expression of ECM, which includes collagen and hyaluronic acid, penetration of TRZ into the center of tumor was limited. Therefore, investigation was conducted on whether TRZ penetration into tumor tissues increases with addition of losartan.
To evaluate effects of losartan, Alexa 647-TRZ (150$\mu$g/100$\mu$l, n = 5) was injected along with 40 mg/kg of losartan (n = 5). FIG. 2A is a representative image of Alexa 647-TRZ in combination with losartan, indicating that losartan enhanced accumulation of TRZ throughout tumor tissues.
As a result of quantifying the fluorescence intensity of TRZ detected at the tumor edge, tumor blood vessels, and the entire tumor in both groups, as shown in FIG. 2B, TRZ used in combination with losartan improved Alexa 647-conjugated TRZ in the entire tumor section (n = 20). For Alexa 647-TRZ, the areas under curve (AUC) for the blood vessels and edges were 3.78 and 1.87 and improved to 4.74 and 3.78 in the case of combination with losartan (FIGS. 2C and 2D).

2-2. Determination of reduction in ECM components by losartan

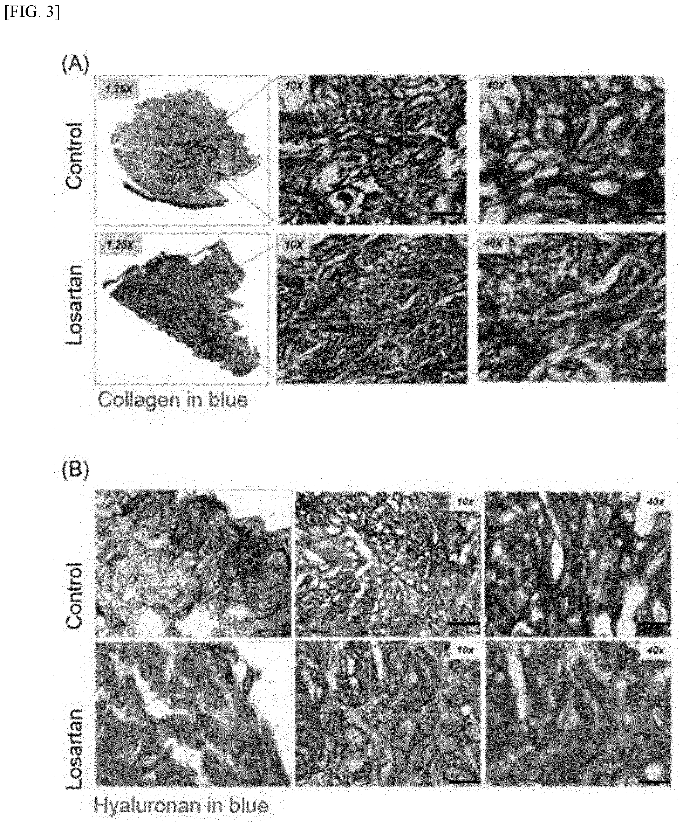

The effect of losartan on ECM components of gastric cancer was evaluated. Collagen and hyaluronan, the main components of ECM, were stained.
It was found via masson trichrome staining that losartan reduced collagen (FIG. 3A). It was found via alcian blue staining that losartan reduced hyaluronan (FIG. 3B). In other words, it was found that losartan significantly reduced the ECM content.

2-3. Identification of $^{64}$Cu-Trastuzumab PET

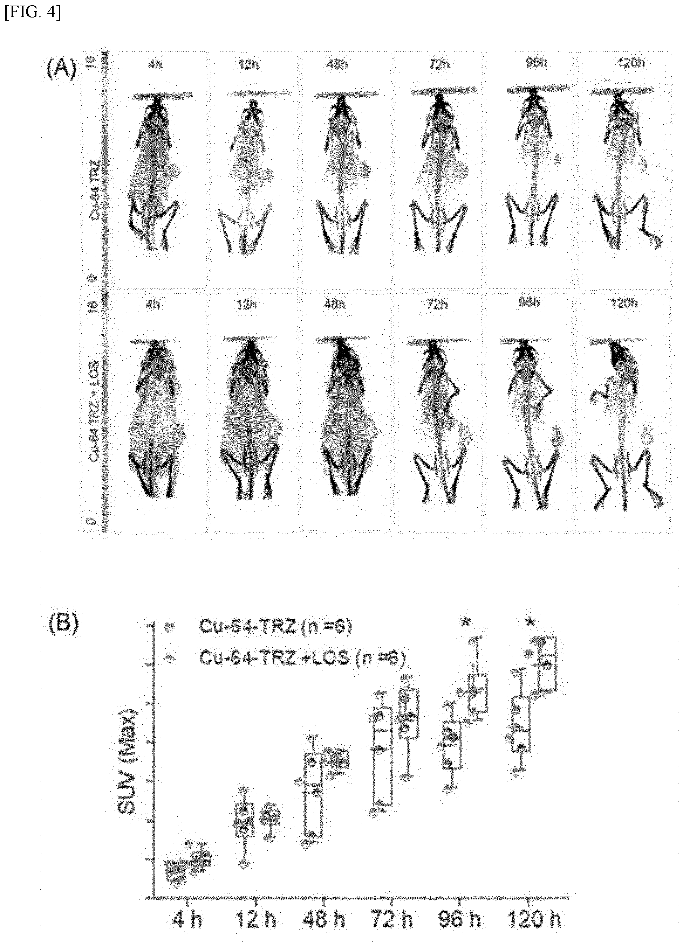

To further evaluate the potential of combination of losartan and $^{64}$Cu-TRZ, positron emission tomography (PET) imaging according to $^{64}$Cu-TRZ or $^{64}$Cu-TRZ in combination with losartan in NCI-N87 xenograft mouse models was performed (FIG. 4A). PET scanning was performed up to 5 days after the first injection. Compared with $^{64}$Cu-TRZ at 96 h and 120 h, the $^{64}$Cu-TRZ in combination with losartan showed a high standard uptake value (SUV), showing statistically significant improvement ($P < 0.05$) (FIG. 4B).

2-4. Identification of effects of slowing tumor growth and improving survival of combined treatment ($^{64}$Cu-TRZ in combination with losartan)

As a result of identifying a treatment effect of co-treatment of $^{64}$Cu-TRZ and losartan as shown in FIG. 5A, the group (Cu-64-TRZ+LOS) treated with $^{64}$Cu-TRZ in combination with losartan slowed tumor growth compared to the group (Cu-64-TRZ) treated with $^{64}$Cu-TRZ. The co-treated group also slowed the tumor growth response compared to the losartan solely treated group (LOS). The data were statistically significant, while the losartan solely treated group showed a tumoricidal response compared to the control.

Reduced tumor growth suggests high absorption of $^{64}$Cu-TRZ by ECM degradation following losartan treatment. Evaluation on survival rate demonstrated the treatment effect of combined RIT using losartan and $^{64}$Cu-TRZ, wherein the median survival rate of $^{64}$Cu-TRZ in combination with losartan was further improved than that of $^{64}$Cu-TRZ ($P < 0.05$) (Fig. 5B). The results indicate that the combined RIT of losartan and $^{64}$Cu-TRZ has therapeutic potential.

2-5. Identification of tumor penetration improvement of cetuximab by losartan

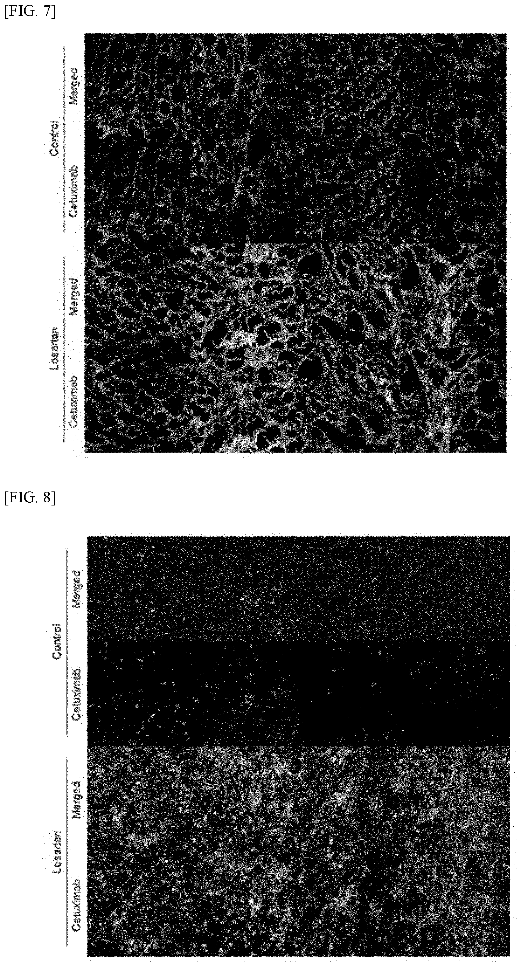

Evaluation was performed on microdistribution of cetuximab over tissues of brain tumor, lung cancer, and colorectal cancer, along with losartan. Investigation was conducted on whether penetration of cetuximab into each tumor tissue increases with addition of losartan. As a result of injecting Alexa 488-cetuximab (150 $\mu$g/100 $\mu$l) along with 60 mg/kg of losartan to evaluate the effect of losartan, accumulation of cetuximab was improved throughout cancerous tumor tissues in brain tumors (FIG. 6), lung cancer (FIG. 7), and colorectal cancer (FIG. 8).

2-6. Identification of tumor penetration improvement of atezolizumab by losartan

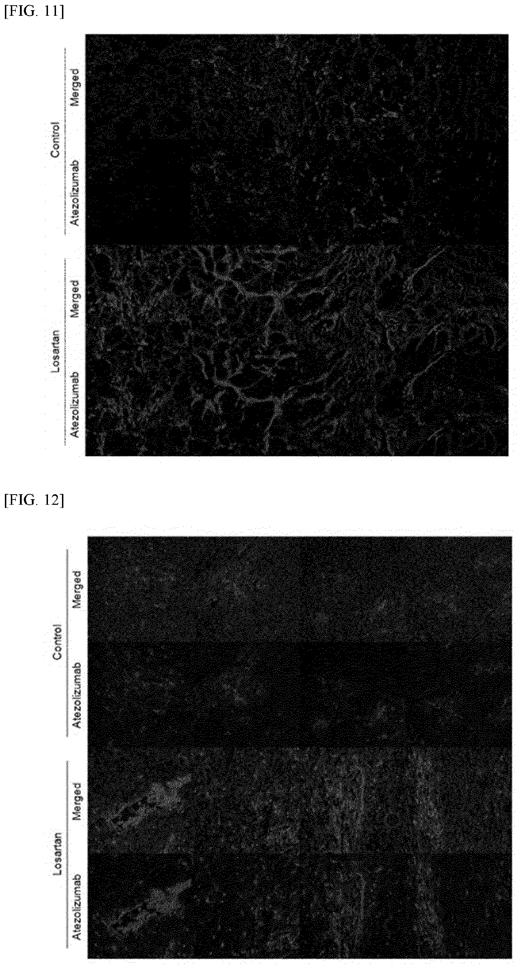

Evaluation was performed on microdistribution of atezolizumab over tissues of pancreatic cancer, brain tumor, lung cancer, and colorectal cancer, along with losartan. Investigation was conducted on whether penetration of atezolizumab into each tumor tissue increases with addition of losartan. As a result of injecting Alexa 647-atezolizumab (150 $\mu$g/100 $\mu$l) along with 60 mg/kg of losartan to evaluate the effect of losartan, accumulation of atezolizumab was improved throughout cancerous tumor tissues in pancreatic cancer (FIG. 9), brain tumor (FIG. 10), lung cancer (FIG. 11), and colorectal cancer (FIG. 12).

2-7. Identification of tumor penetration improvement of rituximab by losartan

Evaluation was performed on microdistribution of rituximab over tissues of B-cell lymphoma along with losartan. Investigation was conducted on whether penetration of rituximab into each tumor tissue increases with addition of losartan. As a result of injecting Alexa 488-rituximab (150 $\mu$g/100 $\mu$l) along with 60 mg/kg of losartan to evaluate the effect of losartan, accumulation of rituximab was improved throughout B-cell lymphoma tissues as shown in FIG. 13.

2-8. Identification of a tumor growth inhibitory effect of combined treatment (irinotecan in combination with losartan)

As a result of identifying the colorectal cancer (CT26) treatment effect in the co-treatment of irinotecan and losartan, as shown in FIG. 14, it was found that the group (LOS+Irinotecan) treated with irinotecan in combination with losartan slowed tumor growth compared to the group treated with irinotecan. The data were statistically significant, while the group co-treated with irinotecan and losartan showed a tumoricidal response compared to the irinotecan solely treated group.

As described above, a specific part of the content of the present disclosure is described in detail, for those of ordinary skill in the art, it is clear that the specific description is only a preferred embodiment, and the scope of the present disclosure is not limited thereby. Accordingly, the substantial scope of the present disclosure will be defined by the appended claims and equivalents thereof.

**Claims**

1. A composition for enhancing tumor penetration of an anticancer drug, comprising losartan as an active ingredient.

2. The composition of claim 1, wherein the composition is a pharmaceutical composition for assisting anticancer treatment.

3. The composition of claim 1, wherein the composition is a pharmaceutical composition for assisting radioimmunotherapy.

4. The composition of claim 1, wherein the anticancer drug is any one of a chemo-anticancer drug, a targeted anticancer drug, or an immuno-anticancer drug labeled or unlabeled with radioisotopes.

5. The composition of claim 4, wherein the chemo-anticancer drug is selected from the group consisting of abemaciclib, everolimus, alpelisib, anastrozole, pamidronic acid, exemestane, capecitabine, eribulin, everolimus, exemestane, toremifene, olaparib, palbociclib, thiotepa, irinotecan, gemcitabine, oxaliplatin, bortezomib, cisplatin, paclitaxel, gefitinib, lapatinib, sorafenib, 5-FU (5-fluorouracil), and doxorubicin.

6. The composition of claim 4, wherein the targeted anticancer drug is selected from the group consisting of abciximab, adalimumab, basiliximab, bezlotoxumab, canakinumab, daclizumab, denosumab, efalizumab, golimumab, inflectra, natalizumab, olaratumab, omalizumab, palivizumab, panitumumab, trastuzumab, pertuzumab, cetuximab, rituximab, tocilizumab, secukinumab, ustekinumab, bevacizumab, and avelumab.

7. The composition of claim 4, wherein the immuno-anticancer drug is selected from the group consisting of an anti-PD-1 antibody, an anti-PD-L1 antibody, and an anti-CTLA-4 antibody.

8. The composition of claim 7, wherein the immuno-anticancer drug is selected from the group consisting of pembrolimab, nivolumab, atezolizumab, durvalumab, and ipilimumab.

9. A pharmaceutical composition for treating a cancer disease, comprising losartan and any one anticancer drug of a chemo-anticancer drug, a targeted anticancer drug, or an immuno-anticancer drug labeled or unlabeled with radioisotopes.

10. The pharmaceutical composition of claim 9, wherein the losartan degrades extracellular matrix (ECM) including collagen and hyaluronic acid to enhance intratumoral penetration of an anticancer drug.

11. The pharmaceutical composition of claim 9, wherein the cancer disease is any one cancer disease selected from the group consisting of gastric cancer, lung cancer, brain tumor, lymphoma, colon cancer, pancreatic cancer, and breast cancer.

[FIG. 1]

[FIG. 2]

[FIG. 3]

(A)

Collagen in blue

(B)

Hyaluronan in blue

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

## INTERNATIONAL SEARCH REPORT

| International application No. |
| --- |
| **PCT/KR2021/014360** |

**A. CLASSIFICATION OF SUBJECT MATTER**

**A61K 31/4178**(2006.01)i; **A61K 39/395**(2006.01)i; **A61K 51/10**(2006.01)i; **A61P 35/00**(2006.01)i; **C07K 16/32**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

A61K 31/4178(2006.01); A61K 31/40(2006.01); A61K 39/395(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 로자탄 (losartan), 항암제 (anti-cancer), 침투 (pentetration), 세포외기질 (ECM, extracellular matrix), 콜라겐 (collagen), 히알루론산 (hyaluronic acid)

**C. DOCUMENTS CONSIDERED TO BE RELEVANT**

| Category* | Citation of document, with indication, where appropriate, of the relevant passages | Relevant to claim No. |
| --- | --- | --- |
| X | ZHAO, Y. et al. Losartan treatment enhances chemotherapy efficacy and reduces ascites in ovarian cancer models by normalizing the tumor stroma. Proceedings of the National Academy of Sciences. 2019, vol. 116, no. 6, pp. 2210-2219.<br>See abstract; and pages 2211-2212 and 2218. | 1-11 |
| X | DIOP-FRIMPONG, B. et al. Losartan inhibits collagen I synthesis and improves the distribution and efficacy of nanotherapeutics in tumors. Proceedings of the National Academy of Sciences. 2011, vol. 108, no. 7, pp. 2909-2914.<br>See abstract; and pages 2909 and 2913. | 1-11 |
| X | CUN, X. et al. A dual strategy to improve the penetration and treatment of breast cancer by combining shrinking nanoparticles with collagen depletion by losartan. Acta biomaterialia. 2016, vol. 31, pp. 186-196.<br>See abstract; page 195; and figure 10. | 1-11 |
| A | JUNG, B.-K. et al. Relaxin-expressing oncolytic adenovirus induces remodeling of physical and immunological aspects of cold tumor to potentiate PD-1 blockade. Journal for immunotherapy of cancer. 2020[August 2020], vol. 8, thesis no. e000763, pp. 1-16.<br>See entire document. | 1-11 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **24 January 2022** | **24 January 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2021/014360** |

**C.     DOCUMENTS CONSIDERED TO BE RELEVANT**

| Category* | Citation of document, with indication, where appropriate, of the relevant passages | Relevant to claim No. |
|---|---|---|
| A | KR 10-2016-0038524 A (KOREA INSTITUTE OF RADIOLOGICAL & MEDICAL SCIENCES) 07 April 2016 (2016-04-07)<br>      See entire document. | 1-11 |

Form PCT/ISA/210 (second sheet) (July 2019)

**INTERNATIONAL SEARCH REPORT**
Information on patent family members

International application No.

**PCT/KR2021/014360**

| Patent document cited in search report | | Publication date (day/month/year) | Patent family member(s) | | Publication date (day/month/year) |
|---|---|---|---|---|---|
| KR 10-2016-0038524 | A | 07 April 2016 | KR 10-1637689 | B1 | 07 July 2016 |

Form PCT/ISA/210 (patent family annex) (July 2019)